# EUROPEAN PATENT SPECIFICATION

(11) **EP 3 006 547 B1**
(45) Date of publication and mention of the grant of the patent: **30.10.2019**
(21) Application number: 14807196.2
(22) Date of filing: 05.06.2014
(51) Int. Cl.: A61K 8/31, A61K 8/58, A61K 8/92, A61Q 3/00, A61K 8/81, A61Q 13/00, A61K 8/11

(54) **FRAGRANCE-CONTAINING CAPSULE AND COSMETIC IN WHICH SAID CAPSULES ARE BLENDED**
DUFTSTOFFHALTIGE KAPSEL UND KOSMETIKUM, IN DEM DIESE KAPSELN GEMISCHT SIND
CAPSULE CONTENANT UNE FRAGRANCE ET PRODUIT COSMÉTIQUE DANS LEQUEL SONT MÉLANGÉES CES CAPSULES

(30) Priority: 07.06.2013 JP 2013120892
(43) Date of publication of application: 13.04.2016
(73) Proprietor: Shiseido Company, Ltd., Chuo-ku Tokyo 104-0061 (JP)
(72) Inventor: SHOJI, Ken, Yokohama Kanagawa 224-8558 (JP); TAGUCHI, Sumie, Yokohama Kanagawa 224-8558 (JP); KANEMARU, Tetsuya, Yokohama Kanagawa 224-8558 (JP); TOYODA, Tomonori, Yokohama Kanagawa 224-8558 (JP); NOMURA, Yasuyuki, Yao-shi Osaka 5810075 (JP); SHIGETA, Hiroaki, Yao-shi Osaka 5810075 (JP)
(74) Representative: Ter Meer Steinmeister & Partner
(86) International application number: PCT/JP2014/064974
(87) International publication number: WO 2014/196602

(56) References cited:
- JP-A- 2003 524 689
- JP-A- 2006 249 326
- US-A1- 2003 125 222
- US-A1- 2012 058 929

## Description

### FIELD OF THE INVENTION

The present invention relates to a fragrance-containing capsule, and in particular, relates to a highly stable and safe fragrance-containing capsule.

### BACKGROUND OF THE INVENTION

In order to provide various impressions on the products such as food/beverage, cosmetics, and detergent, various flavors and fragrances are added. Flavors and fragrances are normally prepared by blending natural fragrances and synthetic fragrances. Flavors and fragrances are generally categorized into top notes, middle notes and base notes depending on the volatility thereof from high to low.

The top note is an important component that determines the first impression of flavor and fragrance and citrusy note and so forth among them is typical to provide freshness, However, since top notes are most volatile, such note disappears in several tens of minutes.

Floral or fruity flavor and fragrance account typically for middle notes. Such middle notes normally yet disappears in several hours.

In Patent Literature 1, a solid fragrance composition containing a fragrance component and powder (for example, poly(methyl methacrylate) ) having a specific absorbed amount of oil is disclosed. It is described that the scents including the top note can last for a long time in such solid fragrance composition. However, the product therefrom is limited to a solid composition, and the scent thereof could not be strengthened greater than that at the beginning of use.

On the other hand, a fragrance encapsulation technology has been developed to prolong scent. A capsule comprises an enclosed core substance and a wall material that forms a wall membrane. As the wall material, polyacrylic ester polymers, urethane polymers, melamine polymers, etc. can be listed.

In Patent Literature 2, for example, an encapsulated fragrance, wherein a fragrance composition having a flash point within the range of 50 to 130 °C is used as the core substance, is described. However, it was not allowed to use such material for a fragrance in cosmetics and so forth from the standpoint of safety because a melamine compound containing formaldehyde is used as the wall material.

If a polymer such as poly(methyl methacrylate) is used as the capsule wall material, the capsule can be used to add fragrance to cosmetics. However, even when the production of a fragrance-enclosed capsule was attempted with the above-described polymer, all the fragrance could not be retained inside the powder and, instead, fragrance-impregnated powder was produced; and further it was difficult to obtain a stable fragrance-containing capsule because of leakage of the fragrance over time, and/or the capsule wall material was prepared by radical polymerization so that the ideally formulated fragrance could not be produced because the fragrance per se was reactive to be integrated into the polymer. When a urethane polymer was used as the wall material, there was a drawback such as lability in water.

In Patent Literature 3, it is described that core shell microcapsules can be applied to fragrance compositions, and the compositions for the wall material (shell) and the fragrance and oil for the core substance (core section) are extensively described. However, stable and safe capsules cannot be guaranteed to be obtained in any combinations because of the above-described reasons and accordingly an improvement has been desired.

The production methods of microcapsules using acrylic polymers as the wall material are described in Patent Literatures 4 and 5. However, the investigation of core substances (enclosed components) and the issues when the fragrance is enclosed are not described.

In Patent Literature 6, fragrance-containing microcapsules are described, wherein geraniol and/or limonene might be used as fragrances.

### Patent Document

Patent literature 1: Japanese unexamined patent publication No. 2010-235746
Patent literature 2: Japanese unexamined patent publication No. 2006-249326
Patent literature 3: International unexamined patent publication No. 2011/064197
Patent literature 4: Japanese unexamined patent publication No. 2003-221578
Patent literature 5: Japanese unexamined patent publication No. H5-15499
Patent literature 6: US 2012/058929 A1 (Laubender Matthias et al.)

### DISCLOSURE OF THE INVENTION

### PROBLEM TO BE SOLVED BY THE INVENTION

The present invention was made in view of the above-described problems of the conventional art, and an object is to provide a highly stable fragrance-containing capsule. In the fragrance-containing capsule of the present invention, a specific fragrance can be stably enclosed inside the wall membrane without fragrance alteration before and after encapsulation. Therefore, well-balanced scents including the top note can be easily provided by smashing the capsule by rubbing etc. at a desired timing for users.

### MEANS TO SOLVE THE PROBLEM

The present inventors have diligently studied to solve the above-described problems and, as a result, found that a stable fragrance-containing capsule can be obtained by using a specific fragrance and oil; and then completed the present invention, accordingly.

That is, the fragrance-containing capsule of the present invention comprises; a core substance consisting of a specific fragrance and oil, and a wall material formed of one or more polymers selected from the group consisting of poly(alkyl-meth-acrylate) and polystyrene, wherein the fragrance is one or more selected from the group consisting of 3-octanol, 2,6-dimethyl-2-heptanol, tetrahydrogeraniol, tetrahydrolinalool, hydroxycitronellol, borneol, cedrol, patchouli alcohol, vetiverol, 4-isopropyl-cyclohexanol, 4-(isopropyl)cyclohexanemethanol, p-tert-butylcyclohexanol, o-tert-butylcyclohexanol, 1-(2-tert-butylcyclohexyloxy)-2-butanol, α,β,2,2,6-pentamethylcyclohexylpropanol, 1-(2,2,6-trimethylcyclohexyl)-3-hexanol, benzyl alcohol, 2-phenylethyl alcohol, phenoxyethyl alcohol, 1-phenylethyl alcohol, anise alcohol, 3-phenylpropyl alcohol, α,α-dimethylbenzyl carbinol, α,α-dimethylphenylethyl carbinol, phenylethylmethylethyl carbinol, 3-methyl-5-phenyl-1-pentanol, thymol, carvacrol, orcinol monomethyl ether, 3,7-dimethyl-7-methoxyoctane-2-ol, 1,8-cineole, 4-acetoxy-3-amyltetrahydropyran, cedryl methyl ether, 1-methoxycyclododecane, 1-methyl-1-methoxycyclododecane, ethoxymethyl-cyclododecyl ether, ambroxan, Gurisaruba, anisole, dimethylhydroquinone, p-cresyl methyl ether, acetanisole, dihydroanethole, diphenyl oxide, phenylethyl isoamyl ether, β-naphthyl methyl ether, β-naphthyl ethyl ether, β-naphthyl isobutyl ether, hexylaldehyde, heptylaldehyde, octylaldehyde, nonylaldehyde, decylaldehyde, undecylaldehyde, 3,5,5-trimethylhexanal, methyloctylacetaldehyde, methylnonylacetaldehyde, hydroxycitronellal, methoxydihydrocitronellal, scentenal, benzaldehyde, phenylacetaldehyde, phenylpropylaldehyde, hydratropic aldehyde, anisaldehyde, p-methylphenylacetaldehyde, cuminaldehyde, cyclamen aldehyde, 3-(p-tert-butylphenyl)-propanal, p-ethyl-2,2-dimethylhydrocinnamaldehyde, 2-methyl-3-(p-methoxyphenyl)-propanal, 4-tert-butyl-α-methyl-hydrocinnamic aldehyde, heliotropine, helional, vanillin, ethylvanillin, octanal glycol acetal, phenylacetaldehyde dimethyl acetal, hydratropic aldehyde dimethyl acetal, phenylacetaldehyde glyceryl acetal, 2-butyl-4,4,6-trimethyl-1,3-dioxane, methyl amyl ketone, ethyl amyl ketone, methyl hexyl ketone, methyl nonyl ketone, camphor, 1-menthone, d-isomenthone, p-tert-butylcyclohexanone, 2-amylcyclopentanone, 2-heptylcyclopentanone, plicatone, 4-cyclohexyl-4-methyl-2-pentanone, 2,2,5-trimethyl-5-pentylcyclopentanone, acetophenone, p-methylacetophenone, benzyl acetone, calone, raspberry ketone, anisyl acetone, zingerone, methyl β-naphthyl ketone, 4-phenyl-4-methyl-2-pentanone, benzophenone, ethyl formate, benzyl formate, phenylethyl formate, ethyl acetate, butyl acetate, isoamyl acetate, hexyl acetate, isononyl acetate, 1-menthyl acetate, n-bornyl acetate, isobornyl acetate, p-t-butylcyclohexyl acetate, o-t-butylcyclohexyl acetate, benzyl acetate, 2-phenylethyl acetate, styralyl acetate, anisyl acetate, p-cresyl acetate, heliotropyl acetate, cedryl acetate, vetiveryl acetate, decahydro-β-naphthyl acetate, ethyl propionate, isoamyl propionate, benzyl propionate, ethyl butyrate, ethyl 2-methylbutyrate, butyl butyrate, isoamyl butyrate, hexyl butyrate, benzyl butyrate, benzyl isobutyrate, phenylethyl isobutyrate, phenoxyethyl isobutyrate, ethyl isovalerate, propyl valerate, benzyl isovalerate, phenylethyl isovalerate, ethyl caproate, ethyl heptanoate, ethyl 2-methylpentanoate, ethyl octanoate, ethyl ketopropionate, isoamyl ketopropionate, ethyl acetoacetate, ethyl levulinate, methyl benzoate, benzyl benzoate, phenylethyl benzoate, methyl phenylacetate, methyl salicylate, methyl anisate, methyl anthranilate, methyl dihydrojasmonate, ethyl 3-methyl-3-phenylglycidate, ethyl 3-phenylglycidate, fructone, fraistone, fruitate, γ-octalactone, coumarin, muscone, cyclopentadecanone, cyclopentadecanolide, and indole.

In the above-described fragrance-containing capsule, it is preferable that the oil is one or more selected from the group consisting of liquid paraffin, ozokerite, squalane, pristane, paraffin, squalene, petrolatum, macadamia nut oil, myristate isopropyl, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, mortierella oil, linear polysiloxanes, and cyclic polysiloxanes.

In the above-described fragrance-containing capsule, it is preferable that polymer comprsing a wall material formed of one or more polymers selected from the group consisting of poly(alkyl (meth)acrylates) and polystyrene.

In the above-described fragrance-containing capsule, the enclosed amount of the fragrance is 1 to 60 mass %, the enclosed amount of the oil is 10 to 89 mass %, and the blending quantity of the wall material is 10 to 30 mass %, with respect to the total amount of the capsule.

In the above-described fragrance-containing capsule, the volume average particle diameter D50 of the capsule is preferably 1 to 100 µm.

The cosmetic of the present invention comprises the above-described fragrance-containing capsules.

In the above-described cosmetic, it is preferable that the blending quantity of alcohol is less than 50 mass %.

### EFFECT OF THE INVENTION

According to the present invention, a highly-stable fragrance-containing capsule without altering the quality of fragrance before and after encapsulation and capable of resurgence of fragrance-scent by rubbing etc. can be provided.

### BRIEF DESCRIPTION OF THE DRAWINGS

Fig. 1 shows an SEM (Scanning Electron Microscope) micrograph of the powder obtained in Production Example 1-1.
Fig. 2 shows an SEM micrograph of the powder obtained in Production Example 2-2.
Fig. 3 shows an SEM micrograph of the fragrance-containing capsule of the present invention (Production Example 3).
Fig. 4 shows the evaluation results for the fragrance-scents on the filter paper.
Fig. 5 shows the evaluation results for the fragrance-scentson the strand.

### BEST MODE FOR CARRYING OUT THE INVENTION

The fragrance-containing capsule of the present invention comprises a core substance consisting of the below-described specific fragrance and oil, and a wall material formed of one or more polymers selected from the group consisting of poly(alkyl -meth-acrylates) and polystyrene.

The fragrance used in core substance of the present invention is one or more fragrances selected from the group consisting of 3-octanol, 2,6-dimethyl-2-heptanol, tetrahydrogeraniol, tetrahydrolinalool, hydroxycitronellol, borneol, cedrol, patchouli alcohol, vetiverol, 4-isopropyl-cyclohexanol, 4-(isopropyl)cyclohexanemethanol, p-tert-butylcyclohexanol, o-tert-butylcyclohexanol, 1-(2-tert-butylcyclohexyloxy)-2-butanol, α,β,2,2,6-pentamethylcyclohexylpropanol, 1-(2,2,6-trimethylcyclohexyl)-3-hexanol, benzyl alcohol, 2-phenylethyl alcohol, phenoxyethyl alcohol, 1-phenylethyl alcohol, anise alcohol, 3-phenylpropyl alcohol, α,α-dimethylbenzyl carbinol, α,α-dimethylphenylethyl carbinol, phenylethylmethylethyl carbinol, 3-methyl-5-phenyl-1-pentanol, thymol, carvacrol, orcinol monomethyl ether, 3,7-dimethyl-7-methoxyoctane-2-ol, 1,8-cineole, 4-acetoxy-3-amyltetrahydropyran, cedryl methyl ether, 1-methoxycyclododecane, 1-methyl-1-methoxycyclododecane, ethoxymethyl-cyclododecyl ether, ambroxan, Gurisaruba, anisole, dimethylhydroquinone, p-cresyl methyl ether, acetanisole, dihydroanethole, diphenyl oxide, phenylethyl isoamyl ether, β-naphthyl methyl ether, β-naphthyl ethyl ether, β-naphthyl isobutyl ether, hexylaldehyde, heptylaldehyde, octylaldehyde, nonylaldehyde, decylaldehyde, undecylaldehyde, 3,5,5-trimethylhexanal, methyloctylacetaldehyde, methylnonylacetaldehyde, hydroxycitronellal, methoxydihydrocitronellal, scentenal, benzaldehyde, phenylacetaldehyde, phenylpropylaldehyde, hydratropic aldehyde, anisaldehyde, p-methylphenylacetaldehyde, cuminaldehyde, cyclamen aldehyde, 3-(p-tert-butylphenyl)-propanal, p-ethyl-2,2-dimethylhydrocinnamaldehyde, 2-methyl-3-(p-methoxyphenyl)-propanal, 4-tert-butyl-α-methyl-hydrocinnamic aldehyde, heliotropine, helional, vanillin, ethylvanillin, octanal glycol acetal, phenylacetaldehyde dimethyl acetal, hydratropic aldehyde dimethyl acetal, phenylacetaldehyde glyceryl acetal, 2-butyl-4,4,6-trimethyl-1,3-dioxane, methyl amyl ketone, ethyl amyl ketone, methyl hexyl ketone, methyl nonyl ketone, camphor, 1-menthone, d-isomenthone, p-tert-butylcyclohexanone, 2-amylcyclopentanone, 2-heptylcyclopentanone, plicatone, 4-cyclohexyl-4-methyl-2-pentanone, 2,2,5-trimethyl-5-pentylcyclopentanone, acetophenone, p-methylacetophenone, benzyl acetone, calone, raspberry ketone, anisyl acetone, zingerone, methyl β-naphthyl ketone, 4-phenyl-4-methyl-2-pentanone, benzophenone, ethyl formate, benzyl formate, phenylethyl formate, ethyl acetate, butyl acetate, isoamyl acetate, hexyl acetate, isononyl acetate, 1-menthyl acetate, n-bornyl acetate, isobornyl acetate, p-t-butylcyclohexyl acetate, o-t-butylcyclohexyl acetate, benzyl acetate, 2-phenylethyl acetate, styralyl acetate, anisyl acetate, p-cresyl acetate, heliotropyl acetate, cedryl acetate, vetiveryl acetate, decahydro-P-naphthyl acetate, ethyl propionate, isoamyl propionate, benzyl propionate, ethyl butyrate, ethyl 2-methylbutyrate, butyl butyrate, isoamyl butyrate, hexyl butyrate, benzyl butyrate, benzyl isobutyrate, phenylethyl isobutyrate, phenoxyethyl isobutyrate, ethyl isovalerate, propyl valerate, benzyl isovalerate, phenylethyl isovalerate, ethyl caproate, ethyl heptanoate, ethyl 2-methylpentanoate, ethyl octanoate, ethyl ketopropionate, isoamyl ketopropionate, ethyl acetoacetate, ethyl levulinate, methyl benzoate, benzyl benzoate, phenylethyl benzoate, methyl phenylacetate, methyl salicylate, methyl anisate, methyl anthranilate, methyl dihydrojasmonate, ethyl 3-methyl-3-phenylglycidate, ethyl 3-phenylglycidate, fructone, fraistone, fruitate, γ-octalactone, coumarin, muscone, cyclopentadecanone, cyclopentadecanolide, and indole.

If the above-described fragrance is used, a stable fragrance-containing capsule can be obtained without the integration of fragrance into the polymer wall material.

The blending quantity of the fragrance is 1 to 60 mass % and more preferably 5 to 40 mass % with respect to the total amount of the capsule. If the blending quantity of the fragrance is too small, the fragrance-scent is too weak and fragrance-scent resurgence maybe hardly sensed by smashing. If the blending quantity of the fragrance is too large, it is difficult to construct the wall material with a polymer and a stable capsule may not be obtained.

Optional oil(s) such as hydrocarbon oil, ester oil, and silicone oil can be used as core substance of the present invention.

Examples of oils include liquid paraffin, ozokerite, squalane, pristane, paraffin, squalene, petrolatum, macadamia nut oil, myristate isopropyl, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, mortierella oil, linear polysiloxanes, and cyclic polysiloxanes.

Examples of linear polysiloxanes include dimethylpolysiloxane, methylphenylpolysiloxane, and diphenylpolysiloxane. Examples of cyclic polysiloxanes include octamethylcyclotetrasiloxane, decamethylcyclopentasiloxane, and dodecamethylcyclohexasiloxane.

As oil of the present invention, squalane and liquid paraffin are preferably used.

The blending quantity of the oil is 10 to 89 mass % with respect to the total amount of the capsule. If the blending quantity of the oil is too small, it is difficult to construct a core-shell structure, and a stable capsule may not be obtained (difficult to retain fragrance). If the blending quantity of the oil is too large, the blending quantity of other components becomes too small and the effect of the present invention may not be obtained.

As polymer of the present invention, poly(alkyl -methacrylates) (for example, poly(methyl methacrylate)) and polystyrene can be used. Furthermore, it is preferable to use a crosslinked polymer rather than a non-crosslinked polymer because more fragrance can be enclosed. Examples of cross-linking agents include ethylene glycol dimethacrylate and divinylbenzene.

In the present invention, the copolymers, wherein another monomer is added as the third component in addition to alkyl methacrylate or styrene and a crosslinking agent, can be used as necessary. Examples of monomers include (meth)acrylic acid, vinyl acetate, acrylamide, acrylonitrile, methacrylonitrile, and vinylidene chloride. The blending ratio of the third component in polymer is preferably 0 to 40 mass %, more preferably 0 to 30 mass % and especially preferably 0 to 20 mass %. Although it depends on the enclosed fragrance and oil, the leakage of fragrance may be likely to occur if the blending ratio of the third component is high.

Furthermore, it is not preferable to use a melamine compound as the polymer because the blending into cosmetics is not allowed from the standpoint of safety.

The blending quantity of the polymer is 10 to 30 mass % with respect to the total amount of the capsule. If the blending quantity of the polymer is too small, the strength of the fragrance-containing capsule is low and the leakage of fragrance component likely takes place. If the blending quantity of the polymer is too large, the capsule strength is too high and the release of fragrance component may be impaired.

The volume average particle diameter D50 of the fragrance-containing capsule of the present invention is preferably 1 to 100 µm and more preferably 5 to 50 µm. If the average particle size is too small, alcohol resistance thereof may be lowered. If the volume average particle diameter is too large, the strength of the fragrance-containing capsule becomes low and the leakage of fragrance component likely takes place, in some cases.

The fragrance-containing capsule of the present invention can be obtained by the ordinary method. For example, a monomer such as methyl methacrylate, oil, etc. are mixed and dissolved, fragrance is added and stirred, and dispersion liquid is obtained. This dispersion liquid is polymerized and then dried; thus the fragrance-containing capsule of the present invention can be obtained.

The amount of loading of each component in the production process and the blending quantity (enclosed amount) of each component in the obtained capsule are approximately the same. Therefore, the fragrance-containing capsule of the present invention can easily be obtained by adjusting the amount of loading in accordance with the blending quantity (enclosed amount) in the intended capsule.

The flavor-and-fragrance-containing capsule of the present invention can be blended in optional product (for example, cosmetic, skin external preparation, air freshener, detergent, fabric softener, bath agent, deodorant, sundries, food and beverages). When the flavor-and-fragrance-containing capsule of the present invention is blended in optional product, the blending quantity of the fragrance-containing capsule is preferably 0.001 to 50 mass % with respect to the total amount of the product. When poly(alkyl -meth-acrylate) is used as a polymer, the fragrance-containing capsule of the present invention is preferably blended in cosmetic.

Examples of cosmetics include skincare cosmetics such as lotion, milky lotion, beauty serum, cream, body lotion, body powder, and body gel, makeup cosmetics such as foundation, pre-makeup, eye shadow, blusher, lipstick, mascara, and face powder, hair cosmetics such as hair mist, hair oil, hair gel, shampoo, rinse, conditioner, treatment, spray, and mousse, and deodorant cosmetics.

In the cosmetic comprising the fragrance-containing capsule, the blending quantity of alcohol is preferably less than 50 mass % and more preferably 45 mass % or less. If the blending quantity of alcohol is too large, the capsules swell and they may not be stable.

### Examples of alcohol include ethanol.

When the fragrance-containing capsule of the present invention is blended in cosmetic, the blending quantity of fragrance-containing capsule is preferably 0.001 to 20 mass % and more preferably 0.3 to 10 mass % with respect to the total amount of the cosmetic.

Furthermore, the product value can be increased by varying the kind of fragrance provided to the cosmetic itself and the kind of fragrance enclosed in the capsule, which is blended into the cosmetic. If the fragrance provided to the cosmetic itself and the fragrance enclosed in the capsule are allowed to be similar, the fragrance is sensed as if it has become stronger by fragrance resurgence when the application position is touched. If the fragrance provided to the cosmetic itself and the fragrance enclosed in the capsule are allowed to be different, dissimilar fragrance is sensed when the application position is touched. Furthermore, if the fragrance provided to the cosmetic itself is omitted and fragrance-containing capsules are blended to the cosmetic, the fragrance is not sensed after the application of the cosmetic; however, the fragrance is sensed when the application position is touched.

### EXAMPLES

The present invention will be further described in the following examples. However, the invention is not limited to these examples. Unless otherwise specified, the blending quantity of each component will be expressed in mass %.

Prior to illustrating the examples, the methods for the evaluation tests used in the present invention will be explained.

### Evaluation (1): Average particle size

As the measurement equipment, a laser diffraction particle size distribution analyzer (HELOS & RODOS, Sympatec GmbH) was used. Each sample was measured by a wet method, and the volume mean diameter D₅₀ value was set as the average particle size.

### Evaluation (2): Capsulation evaluation

Each sample was observed visually and/or observed by SEM. Subsequently, the fragrance of each sample was evaluated after rubbing. Here, rubbing is performed twice by the hand with an equal force.
A: Sample did not aggregate and powder could be isolated. In the SEM observation, cavity could be observed on the cross section; thus capsule was formed.
B: Sample did not aggregate and powder could be isolated. In the SEM observation, no cavity was observed on the cross section; thus it was powdery.
C: Sample did not aggregate; however, the capsule strength was low when it was isolated, and the outflow of the enclosed material was observed.
D: Sample aggregated and could not be isolated.

### Evaluation (3): Fragrance-scents evaluation

Two expert panelists evaluated the fragrance of each sample (as it is, or after rubbing) based on the below-described scoring criteria. The evaluation was based on the average of the scores given by the two panelists. Here, rubbing is performed twice by the hand with an equal force.
6 points: very strong
5 points: strong
4 points: neither strong nor weak
3 points: weak
2 points: recognizable
1 point: detectable
0 point: not fragrant

### Evaluation (4): Alcohol resistance

After the elapse of 4 weeks from the time of the preparation of each sample, alcohol resistance (appearance/fragrance-scent) of the sample was evaluated based on the below-described evaluation criteria. Here, rubbing is performed twice by the hand with an equal force.

### (Appearance)

A: No aggregation, no solidification, and no oil droplet were observed.
B: No aggregation and no solidification were observed; however, oil droplets were observed.
C: Aggregation, solidification, and oil droplets were observed.

### (Fragrance-scent)

A: A sample was applied on a filter paper; fragrance-scent was strengthened by rubbing.
B: A sample was applied on a filter paper; fragrance-scent was slightly strengthened by rubbing.
C: The fragrance-scent was already strong before application, and the strength of fragrance did not change either by the application of sample on a filter paper or by rubbing.

The present inventors attempted the production of a capsule wherein fragrance is contained as the core substance and poly(methyl methacrylate) was used as the wall material. That is, the present inventors attempted the production of capsules with the compositions shown in the below Table 1 by the ordinary method (below-described production method). Then, each sample was evaluated by the above-described evaluation methods for evaluation items (1) to (3). The result is shown in Table 1.

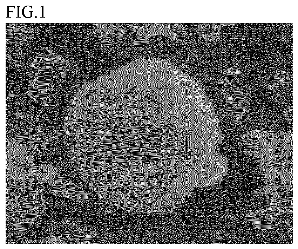

In addition, an SEM micrograph for Production Example 1-1 is shown in Fig. 1.

### • Production method

### (Production Example 1-1)

An oil mixture was prepared by uniformly dissolving 66.5 g of methyl methacrylate, 3.5 g of ethylene glycol dimethacrylate, 0.3 g of 2,2'-azobis(2,4-dimethylvaleronitrile), 15 g of squalane, and 15 g of fragrance (limonene). An aqueous mixture was prepared by uniformly mixing 500 g of water, and 4 g of methyl cellulose.

Subsequently, an aqueous dispersion was prepared by suspending the oil mixture and the aqueous mixture with a homomixer, and it was loaded into an autoclave and reacted under the conditions of 60 °C and 0.2 MPa for 12 hours. The reaction product was filtered and dried to obtain particles.

### (Production Example 1-2)

Production Example 1-2 was produced in the same way as the above Production Example 1-1 with the blending quantities of Table 1.

**[Table 1]**

| Production Example | | 1-1 | 1-2 |
|---|---|---|---|
| Wall material | Crosslinked poly(methyl methacrylate) | 70 | 55 |
| Core substance | Fragrance (Limonene) | 15 | 40 |
| | Squalane | 15 | 5 |
| Average particle size (µm) | | 8 | 10 |
| Capsulation evaluation | | B | D |
| Fragrance evaluation | | 0 | - |
| Fragrance evaluation (after rubbing) | | 0 | - |

According to Production Example 1-1 and Fig. 1, it was found that the capsulation is not possible with this composition and powder was obtained. In addition, limonene reacted with poly(methyl methacrylate) and was integrated in the wall material, the fragrance of limonene was not sensed as it is, and the fragrance of limonene was not sensed even by rubbing.

According to Production Example 1-2, it was found that when the blending quantity of the enclosed material (limonene) was increased, the isolation of powder turned out to be impossible.

Therefore, the present inventors carried out an investigation using different fragrance as core substance. That is, the production of capsules with the compositions shown in the below Table 2 was attempted in the same way as the above Production Example 1-1 (with the blending quantities of Table 2). Then, each sample was evaluated by the above-described evaluation methods for evaluation items (1) to (3). The results are shown in Table 2.

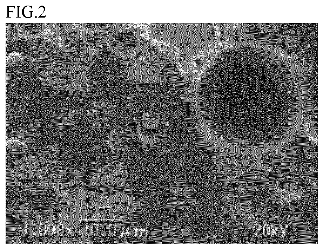

In addition, an SEM micrograph of the cross section for Production Example 2-2 is shown in Fig. 2.

**[Table 2]**

| Production Example | | 2-1 | 2-2 | 2-3 | 2-4 |
|---|---|---|---|---|---|
| Wall material | Crosslinked poly(methyl methacrylate) | 75 | 70 | 65 | 60 |
| Core substance | Fragrance (Ethyl caproate) | 25 | 30 | 35 | 40 |
| Average particle size (µm) | | 10 | 10 | 10 | 8 |
| Capsulation evaluation | | B | B | D | D |
| Fragrance evaluation | | 5 | 5 | - | - |
| Fragrance evaluation (after rubbing) | | 5 | 5 | - | - |

According to Production Examples 2-1, 2-2, and Fig. 2, when the fragrance (ethyl caproate) was used alone as the core substance, a capsule could not be obtained and ethyl caproate-impregnated powder was obtained. However, it was not integrated in the wall material because of proving the presence of fragrance-scent.

Since generation of impregnated powder is a phenomenon when the polarity difference between the enclosed material and the wall material is small, the present inventors conceived that it was necessary to lower the polarity of the material to be enclosed.

Impregnated powder is not in a so-called core-shell structure, and it indicates the state in which fragrance is penetrated into powder. In this state, the leakage of fragrance cannot be prevented and the resurgent effect of fragrance can be hardly obtained.

Therefore, the present inventors attempted the production of a stable capsule by using ethyl caproate as the core substance. At first, the present inventors carried out an investigation by using poly(methyl methacrylate) as the wall material and an oil in addition to the fragrance as the core substance. That is, the present inventors attempted the production of capsules with the compositions shown in the below Table 3 by the ordinary method (below-described production method). Then, each sample was evaluated by the above-described evaluation methods for evaluation items (1) to (3). The result is shown in Table 3.

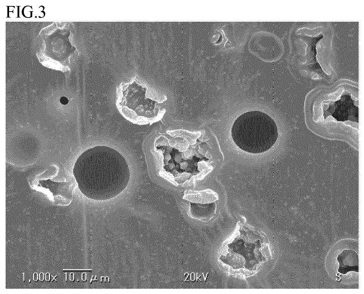

In addition, an SEM micrograph of the cross section for Production Example 3 is shown in Fig. 3.

### • Production method (Production Example 3)

An oil mixture was prepared by uniformly dissolving 20 g of methyl methacrylate, 0.2 g of 2,2'-azobis(2,4-dimethylvaleronitrile), 65 g of squalane, and 15 g of ethyl caproate. An aqueous mixture was prepared by uniformly mixing 400 g of water, 10 g of colloidal silica, and 1 g of diethanolamine-adipic acid condensation product and adjusting to pH 3.

Subsequently, an aqueous dispersion was prepared by suspending the oil mixture and the aqueous mixture with a homomixer, and it was loaded into an autoclave and reacted under the conditions of 60 °C and 0.2 MPa for 12 hours. The reaction product was filtered and dried to obtain particles.

**[Table 3]**

| Production Example | | 3 |
|---|---|---|
| Wall material | Non-crosslinked poly(methyl methacrylate) | 20 |
| Core substance | Fragrance (Ethyl caproate) | 15 |
| | Squalane | 65 |
| Average particle size (µm) | | 25 |
| Capsulation evaluation | | A |
| Fragrance evaluation | | 3 |
| Fragrance evaluation (after rubbing) | | 6 |

According to Production Example 3 and Fig. 3, when not only a fragrance but also an oil (squalane) was used as the core substance to lower the polarity of the enclosed material, a polymer wall could be constructed and a cavity was observed on the cross section; thus it was understood that an ethyl caproate-containing capsule was obtained. Because the fragrance became strong by rubbing the capsule, it was understood that ethyl caproate was stably enclosed in the capsule of Production Example 3.

Accordingly, in the fragrance-containing capsule of the present invention, it is necessary to use a fragrance represented by ethyl caproate and an oil as the core substance.

Subsequently, the present inventors carried out an investigation concerning the enclosed fragrance in the capsule wherein oil and fragrance are used as the core substance. That is, the present inventors attempted the production of capsules with the compositions shown in the below Table 4 blending each fragrance shown in below Table 5 by the ordinary method (below-described production method). Then, each sample was evaluated by the above-described evaluation method for evaluation item (2). The results are shown in Table 5.

### • Production method (Production Example 4)

An oil mixture was prepared by uniformly dissolving 23.7 g of methyl methacrylate, 1.3 g of ethylene glycol dimethacrylate, 0.2 g of 2,2'-azobis(2,4-dimethylvaleronitrile), 65 g of liquid paraffin, and 15 g of each fragrance shown in Table 5. An aqueous mixture was prepared by uniformly mixing 400 g of water, 10 g of colloidal silica, and 1 g of diethanolamine-adipic acid condensation product and adjusting to pH 3.

Subsequently, an aqueous dispersion was prepared by suspending the oil mixture and the aqueous mixture with a homomixer, and it was loaded into an autoclave and reacted under the conditions of 60 °C and 0.2 MPa for 12 hours. The reaction product was filtered and dried to obtain particles.

**(Table 4)**

| • Core substance | |
|---|---|
| Each fragrance shown in Table 5 | 15 mass % |
| Liquid paraffin | 60 |

| • Wall material | |
|---|---|
| Crosslinked poly(methyl methacrylate) | 25 |

**[Table 5-1]**

| Fragrance | Capsulation evaluation |
|---|---|
| limonene | B |
| cis-3-hexenol | B |
| geraniol | B |
| allyl hexanoate | B |
| 3-octanol | A |
| 2,6-dimethyl-2-heptanol | A |
| tetrahydrogeraniol | A |
| tetrahydrolinalool | A |
| hydroxycitronellol | A |
| borneol | A |
| cedrol | A |
| patchouli alcohol | A |
| vetiverol | A |
| 4-isopropyl-cyclohexanol | A |
| 4-(isopropyl)cyclohexanemethanol | A |
| p-tert-butylcyclohexanol | A |
| 1-(2,2,6-trimethylcyclohexyl)-3-hexanol | A |
| α,α-dimethylbenzyl carbinol | A |
| α,α-dimethylphenylethyl carbinol | A |
| 4-acetoxy-3 -amyltetrahydropyran | A |
| cedryl methyl ether | A |
| 1 -methoxycyclododecane | A |
| Gurisaruba | A |
| anisole | A |
| dimethylhydroquinone | A |
| p-cresyl methyl ether | A |
| acetanisole | A |
| dihydroanethole | A |

**[Table 5-2]**

| Fragrance | Capsulation evaluation |
|---|---|
| diphenyl oxide | A |
| phenylethyl isoamyl ether | A |
| β-naphthyl methyl ether | A |
| β-naphthyl ethyl ether | A |
| β-naphthyl isobutyl ether | A |
| hexylaldehyde | A |
| heptylaldehyde | A |
| octylaldehyde | A |
| p-methylphenylacetaldehyde | A |
| cuminaldehyde | A |
| 3-(p-tert-butylphenyl)-propanal | A |
| 2-methyl-3-(p-methoxyphenyl)-propanal | A |
| phenylacetaldehyde dimethyl acetal | A |
| hydratropic aldehyde dimethyl acetal | A |
| methyl amyl ketone | A |
| ethyl amyl ketone | A |
| methyl hexyl ketone | A |
| methyl nonyl ketone | A |
| camphor | A |
| 1-menthone | A |
| d-isomenthone | A |
| 2-amylcyclopentanone | A |
| 2-heptylcyclopentanone | A |
| plicatone | A |
| 4-cyclohexyl-4-methyl-2-pentanone | A |
| 2,2,5-trimethyl-5-pentylcyclopentanone | A |
| anisyl acetone | A |
| zingerone | A |
| methyl β-naphthyl ketone | A |

**[Table 5-3]**

| Fragrance | Capsulation evaluation |
|---|---|
| benzophenone | A |
| ethyl formate | A |
| benzyl formate | A |
| phenylethyl formate | A |
| ethyl acetate | A |
| p-t-butylcyclohexyl acetate | A |
| 2-phenylethyl acetate | A |
| styralyl acetate | A |
| anisyl acetate | A |
| p-cresyl acetate | A |
| heliotropyl acetate | A |
| cedryl acetate | A |
| vetiveryl acetate | A |
| decahydro-β-naphthyl acetate | A |
| hexyl butyrate | A |
| benzyl butyrate | A |
| benzyl isobutyrate | A |
| phenylethyl isobutyrate | A |
| phenoxyethyl isobutyrate | A |
| ethyl isovalerate | A |
| propyl valerate | A |
| benzyl isovalerate | A |
| phenylethyl isovalerate | A |
| ethyl caproate | A |
| methyl phenylacetate | A |
| methyl salicylate | A |
| methyl anisate | A |
| methyl anthranilate | A |
| methyl dihydrojasmonate | A |

**[Table 5-4]**

| Fragrance | Capsulation evaluation |
|---|---|
| coumarin | A |
| muscone | A |
| indole | A |
| benzyl alcohol | A |
| 2-phenylethyl alcohol | A |
| phenoxyethyl alcohol | A |
| 1-phenylethyl alcohol | A |
| anise alcohol | A |
| 3-phenylpropyl alcohol | A |
| thymol | A |
| carvacrol | A |
| orcinol monomethyl ether | A |
| o-tert-butylcyclohexanol | A |
| 1-(2-tert-butylcyclohexyloxy)-2-butanol | A |
| α,β,2,2,6-pentamethylcyclohexylpropanol | A |
| phenylethylmethylethyl carbinol | A |
| 3-methyl-5-phenyl-1-pentanol | A |
| 3,7-dimethyl-7-methoxyoctane-2-ol | A |
| 1-methyl-1-methoxycyclododecane | A |
| 1,8-cineole | A |
| ambroxan | A |
| nonylaldehyde | A |
| decylaldehyde | A |
| undecylaldehyde | A |
| 3,5,5-trimethylhexanal | A |
| methyloctylacetaldehyde | A |
| methylnonylacetaldehyde | A |
| hydroxycitronellal | A |
| methoxydihydrocitronellal | A |

**[Table 5-5]**

| Fragrance | Capsulation evaluation |
|---|---|
| scentenal | A |
| benzaldehyde | A |
| phenylacetaldehyde | A |
| phenylpropylaldehyde | A |
| hydratropic aldehyde | A |
| anisaldehyde | A |
| p-ethyl-2,2-dimethylhydrocinnamaldehyde | A |
| cyclamen aldehyde | A |
| ethoxymethyl-cyclododecyl ether | A |
| 4-tert-butyl-α-methyl-hydrocinnamic aldehyde | A |
| phenylacetaldehyde glyceryl acetal | A |
| 2-butyl-4,4,6-trimethyl-1,3-dioxane | A |
| heliotropine | A |
| helional | A |
| vanillin | A |
| ethylvanillin | A |
| octanal glycol acetal | A |
| acetophenone | A |
| p-methylacetophenone | A |
| benzyl acetone | A |
| calone | A |
| raspberry ketone | A |
| p-tert-butylcyclohexanone | A |
| 4-phenyl-4-methyl-2-pentanone | A |
| butyl acetate | A |
| isoamyl acetate | A |
| hexyl acetate | A |
| isononyl acetate | A |
| 1-menthyl acetate | A |

**[Table 5-6]**

| Fragrance | Capsulation evaluation |
|---|---|
| n-bornyl acetate | A |
| isobornyl acetate | A |
| benzyl acetate | A |
| o-t-butylcyclohexyl acetate | A |
| ethyl propionate | A |
| isoamyl propionate | A |
| benzyl propionate | A |
| ethyl butyrate | A |
| ethyl 2-methylbutyrate | A |
| butyl butyrate | A |
| isoamyl butyrate | A |
| ethyl 3-methyl-3-phenylglycidate | A |
| ethyl heptanoate | A |
| ethyl 2-methylpentanoate | A |
| ethyl octanoate | A |
| ethyl ketopropionate | A |
| isoamyl ketopropionate | A |
| ethyl acetoacetate | A |
| ethyl levulinate | A |
| methyl benzoate | A |
| benzyl benzoate | A |
| phenylethyl benzoate | A |
| ethyl 3-phenylglycidate | A |
| fructone | A |
| fraistone | A |
| fruitate | A |
| γ-octalactone | A |
| cyclopentadecanone | A |
| cyclopentadecanolide | A |

From Table 5, it is seen that it is necessary to select a to-be-enclosed fragrance to obtain a stable fragrance-containing capsule. When the enclosure of the fragrance such as limonene, cis-3-hexenol, geraniol, or allyl hexanoate was tried, the fragrance reacted with the wall material and it was integrated therein; thus a fragrance-containing capsule could not be obtained and it became powdery.

Accordingly, it is necessary that the fragrance-containing capsule of the present invention comprises a core substance comprising fragrance and oil, and a wall material formed of one or more polymers selected from the group consisting of poly(alkyl (meth)acrylates) and polystyrene, wherein the fragrance is one or more selected from the group consisting of 3-octanol, 2,6-dimethyl-2-heptanol, tetrahydrogeraniol, tetrahydrolinalool, hydroxycitronellol, borneol, cedrol, patchouli alcohol, vetiverol, 4-isopropyl-cyclohexanol, 4-(isopropyl)cyclohexanemethanol, p-tert-butylcyclohexanol, o-tert-butylcyclohexanol, 1-(2-tert-butylcyclohexyloxy)-2-butanol, α,β,2,2,6-pentamethylcyclohexylpropanol, 1-(2,2,6-trimethylcyclohexyl)-3-hexanol, benzyl alcohol, 2-phenylethyl alcohol, phenoxyethyl alcohol, 1-phenylethyl alcohol, anise alcohol, 3-phenylpropyl alcohol, α,α-dimethylbenzyl carbinol, α,α-dimethylphenylethyl carbinol, phenylethylmethylethyl carbinol, 3-methyl-5-phenyl-1-pentanol, thymol, carvacrol, orcinol monomethyl ether, 3,7-dimethyl-7-methoxyoctane-2-ol, 1,8-cineole, 4-acetoxy-3-amyltetrahydropyran, cedryl methyl ether, 1-methoxycyclododecane, 1-methyl-1-methoxycyclododecane, ethoxymethyl-cyclododecyl ether, ambroxan, Gurisaruba, anisole, dimethylhydroquinone, p-cresyl methyl ether, acetanisole, dihydroanethole, diphenyl oxide, phenylethyl isoamyl ether, β-naphthyl methyl ether, β-naphthyl ethyl ether, β-naphthyl isobutyl ether, hexylaldehyde, heptylaldehyde, octylaldehyde, nonylaldehyde, decylaldehyde, undecylaldehyde, 3,5,5-trimethylhexanal, methyloctylacetaldehyde, methylnonylacetaldehyde, hydroxycitronellal, methoxydihydrocitronellal, scentenal, benzaldehyde, phenylacetaldehyde, phenylpropylaldehyde, hydratropic aldehyde, anisaldehyde, p-methylphenylacetaldehyde, cuminaldehyde, cyclamen aldehyde, 3-(p-tert-butylphenyl)-propanal, p-ethyl-2,2-dimethylhydrocinnamaldehyde, 2-methyl-3-(p-methoxyphenyl)-propanal, 4-tert-butyl-α-methyl-hydrocinnamic aldehyde, heliotropine, helional, vanillin, ethylvanillin, octanal glycol acetal, phenylacetaldehyde dimethyl acetal, hydratropic aldehyde dimethyl acetal, phenylacetaldehyde glyceryl acetal, 2-butyl-4,4,6-trimethyl-1,3-dioxane, methyl amyl ketone, ethyl amyl ketone, methyl hexyl ketone, methyl nonyl ketone, camphor, 1-menthone, d-isomenthone, p-tert-butylcyclohexanone, 2-amylcyclopentanone, 2-heptylcyclopentanone, plicatone, 4-cyclohexyl-4-methyl-2-pentanone, 2,2,5-trimethyl-5-pentylcyclopentanone, acetophenone, p-methylacetophenone, benzyl acetone, calone, raspberry ketone, anisyl acetone, zingerone, methyl β-naphthyl ketone, 4-phenyl-4-methyl-2-pentanone, benzophenone, ethyl formate, benzyl formate, phenylethyl formate, ethyl acetate, butyl acetate, isoamyl acetate, hexyl acetate, isononyl acetate, 1-menthyl acetate, n-bornyl acetate, isobornyl acetate, p-t-butylcyclohexyl acetate, o-t-butylcyclohexyl acetate, benzyl acetate, 2-phenylethyl acetate, styralyl acetate, anisyl acetate, p-cresyl acetate, heliotropyl acetate, cedryl acetate, vetiveryl acetate, decahydro-β-naphthyl acetate, ethyl propionate, isoamyl propionate, benzyl propionate, ethyl butyrate, ethyl 2-methylbutyrate, butyl butyrate, isoamyl butyrate, hexyl butyrate, benzyl butyrate, benzyl isobutyrate, phenylethyl isobutyrate, phenoxyethyl isobutyrate, ethyl isovalerate, propyl valerate, benzyl isovalerate, phenylethyl isovalerate, ethyl caproate, ethyl heptanoate, ethyl 2-methylpentanoate, ethyl octanoate, ethyl ketopropionate, isoamyl ketopropionate, ethyl acetoacetate, ethyl levulinate, methyl benzoate, benzyl benzoate, phenylethyl benzoate, methyl phenylacetate, methyl salicylate, methyl anisate, methyl anthranilate, methyl dihydrojasmonate, ethyl 3-methyl-3-phenylglycidate, ethyl 3-phenylglycidate, fructone, fraistone, fruitate, γ-octalactone, coumarin, muscone, cyclopentadecanone, cyclopentadecanolide, and indole.

The fragrance-containing capsule of the present invention can stably enclose fragrance so that as if just applied fragrance even with the top note can easily be realized by rubbing.

Subsequently, the present inventors investigated the blending quantity of each component in the capsule by using the mixed fragrance wherein the above-described specific fragrances of the present invention are blended. That is, after the preparation of the mixed fragrance A shown below, the present inventors attempted the production of capsules with the compositions shown in the below Table 6 by the ordinary method (below-described production method). Then, each sample was evaluated by the above-described evaluation methods for evaluation items (1) and (2). The results are shown in Table 6.

### • Production method

### (Production Example 4-1)

An oil mixture was prepared by uniformly dissolving 23.7 g of methyl methacrylate, 1.3 g of ethylene glycol dimethacrylate, 0.2 g of 2,2'-azobis(2,4-dimethylvaleronitrile), 65 g of squalane, and 10 g of mixed fragrance A. An aqueous mixture was prepared by uniformly mixing 400 g of water, 10 g of colloidal silica, and 1 g of diethanolamine-adipic acid condensation product and adjusting to pH 3.

Subsequently, an aqueous dispersion was prepared by suspending the oil mixture and the aqueous mixture with a homomixer, and it was loaded into an autoclave and reacted under the conditions of 60 °C and 0.2 MPa for 12 hours. The reaction product was filtered and dried to obtain particles.

### (Production Examples 4-2 to 4-7)

Production Examples 4-2 to 4-7 were produced in the same way as the above Production Example 4-1 with the blending quantities of Table 6.

**<Mixed fragrance A>**

| Octanal glycol acetal | 2 mass % |
|---|---|
| Methyloctylacetaldehyde | 1 |
| Octylaldehyde | 5 |
| Methyl benzoate | 3 |
| Ethyl heptanoate, | 2 |
| Hexyl butyrate | 2 |
| Hexyl acetate | 5 |
| 2-amylcyclopentanone | 1 |
| Hexylaldehyde | 3 |
| Phenylacetaldehyde | 3 |
| Phenoxyethyl alcohol | 5 |
| 2-phenylethyl alcohol | 7 |
| α,α-dimethylbenzyl carbinol | 2 |
| Benzyl benzoate | 2 |
| p-cresyl acetate | 2 |
| Diphenyl oxide | 2 |
| Tetrahydrogeraniol | 3 |
| Phenylethyl isoamyl ether | 2 |
| 1-menthone | 3 |
| Camphor | 2 |
| 2-butyl-4,4,6-trimethyl-1,3-dioxane | 2 |
| 1,8-cineole | 1 |
| Benzyl acetate | 6 |
| 4-acetoxy-3-amyltetrahydropyran | 1 |
| Ethyl 2-methylpentanoate | 1 |
| Cyclopentadecanolide | 1 |
| Cyclopentadecanone | 1 |
| 1-methyl-1-methoxycyclododecane | 2 |
| 4-cyclohexyl-4-methyl-2-pentanone | 1 |
| p-tert-butylcyclohexanone | 3 |
| Cedryl acetate | 2 |
| Cedrol | 2 |
| Methyl dihydrojasmonate | 20 |

**[Table 6]**

| Production Example | | 4-1 | 4-2 | 4-3 | 4-4 | 4-6 | 4-7 |
|---|---|---|---|---|---|---|---|
| Wall material | Crosslinked poly(methyl methacrylate) | 25 | 25 | 25 | 25 | 30 | 9 |
| Core substance | Mixed fragrance A | 10 | 15 | 20 | 50 | 65 | 50 |
| | Squalane | 65 | 60 | 55 | - | - | - |
| | Liquid paraffin | - | - | - | 25 | 5 | 41 |
| Average particle size (µm) | | 23 | 25 | 24 | 23 | 26 | 25 |
| Capsulation evaluation | | A | A | A | A | B | C |

Even when the fragrance was varied, capsules wherein 15% or more of the fragrance was enclosed could be isolated. Up to 60% of the enclosed fragrance, a constructed polymer wall and a cavity were observed in the SEM observation of the capsule cross section.

It was found, by comparing with Production Example 3, that when crosslinked poly(methyl methacrylate) was used as the wall material, more fragrance could be enclosed.

From Production Example 4-6, if the blending quantity of oil was small and the blending quantity of fragrance was large, the construction of a polymer wall could not be completed and impregnated powder was formed. From Production Example 4-7, if the blending quantity of polymer was small, the strength was low and it could not be handled as a capsule.

As a result of the investigation by the present inventors, it was found that the blending quantity of fragrance is preferably 1 to 60 mass%, the blending quantity of oil is preferably 10 to 89 mass%, and the blending quantity of the wall material is preferably 10 to 30 mass% with respect to the total amount of the fragrance-containing capsule of the present invention.

Subsequently, the present inventors investigated wall materials other than poly(alkyl (meth)acrylate). That is, the observation was carried out with SEM after capsules were produced by the below-described production method.

### • Production method (Production Example 5)

An oil mixture was prepared by uniformly dissolving 23.7 g of styrene, 1.3 g of divinylbenzene, 0.2 g of 2,2'-azobis(2,4-dimethylvaleronitrile), 65 g of squalane, and 10 g of mixed fragrance A. An aqueous mixture was prepared by uniformly mixing 400 g of water, 10 g of colloidal silica, and 1 g of diethanolamine-adipic acid condensation product and adjusting to pH 3.

Subsequently, an aqueous dispersion was prepared by suspending the oil mixture and the aqueous mixture with a homomixer, and it was loaded into an autoclave and reacted under the conditions of 60 °C and 0.2 MPa for 12 hours. The reaction product was filtered and dried to obtain particles.

The average particle size of the capsule was 29 µm, and a constructed polymer wall and a cavity were observed in the SEM observation of the capsule cross section. Because the fragrance was strengthened by rubbing, it was understood that the fragrance was stably enclosed.

### • Production method (Production Example 6)

An oil mixture was prepared by uniformly dissolving 14.2 g of methyl methacrylate,9.5 g of styrene, 1.3 g of ethylene glycol dimethacrylate, 0.2 g of 2,2'-azobis(2,4-dimethylvaleronitrile), 65 g of liquid paraffin, and 10 g of mixed fragrance A. An aqueous mixture was prepared by uniformly mixing 400 g of water, 10 g of colloidal silica, and 1 g of diethanolamine-adipic acid condensation product and adjusting to pH 3.

Subsequently, an aqueous dispersion was prepared by suspending the oil mixture and the aqueous mixture with a homomixer, and it was loaded into an autoclave and reacted under the conditions of 60 °C and 0.2 MPa for 12 hours. The reaction product was filtered and dried to obtain particles.

The average particle size of the capsule was 25 µm, and a constructed polymer wall and a cavity were observed in the SEM observation of the capsule cross section. Because the fragrance was strengthened by rubbing, it was understood that the fragrance was stably enclosed.

From the results of these investigations, it was found that fragrance-containing capsules can be obtained not only when a poly(alkyl (meth)acrylate) is used as the wall material but also when polystyrene or a copolymer of alkyl (meth)acrylate and styrene is used.

Subsequently, the fragrance when the fragrance-containing capsule of the present invention is blended into cosmetics was investigated. That is, a cosmetic (hair mist) containing the fragrance-containing capsule obtained in the above Production Example 3 was prepared, in the compositions shown in the below Table 7, by the ordinary method. The value in [square brackets] in Table 7 is the blending quantity of fragrance (with respect to the total amount of the cosmetic).

Then, each sample was evaluated by the above-described evaluation method for evaluation item (3). The evaluation was carried out after being allowed to leave for 5 minutes, 15 minutes, 30 minutes, 1 hour, and 2 hours after the application of each sample on a filter paper (amount of application: 0.1 ml) or on a strand (amount of application: 0.2 ml).

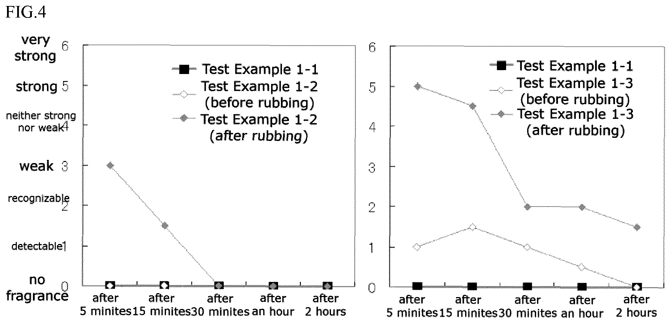

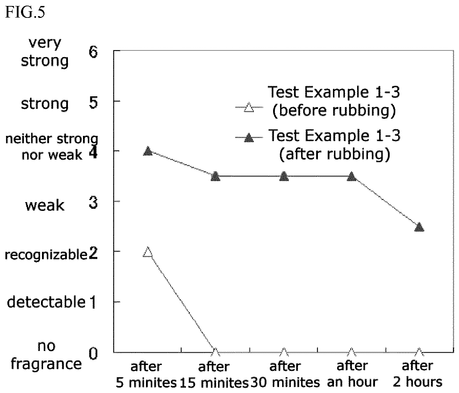

The evaluation results in filter paper and strand are shown in Fig. 4 and Fig. 5, respectively.

**[Table 7]**

| Test Example | 1-1 | 1-2 | 1-3 |
|---|---|---|---|
| Fragrance | 0.2 | - | - |
| Fragrance-containing capsule in Production Example 3 (Enclosed amount of fragrance: 15%) | - | 0.2[0.03] | 1.2[0.18] |
| Ethanol | 8 | 8 | 8 |
| Glycerin | 2 | 2 | 2 |
| Stearyltrimethylammonium chloride (25%) | 0.6 | 0.6 | 0.6 |
| Ion-exchanged water | balance | balance | balance |

It is seen from Fig. 4 that even if the fragrance alone providing no residual scent, the fragrance-scent could be resurged by encapsulated-blending and rubbing. From Fig. 4, it is seen that even when the blending quantity of fragrance-containing capsules is very small, the fragrance-containing capsules can resurge the fragrance by rubbing. However, it is seen from Fig. 4 that it is better to blend a suitable amount of fragrance-containing capsules since a strong fragrance can be resurged every repeated rubbing over a long period of time.

Accordingly, when the fragrance-containing capsule of the present invention is blended in cosmetic, the blending quantity of fragrance-containing capsule is preferably 0.3 mass % or more with respect to the total amount of the cosmetic.

It is seen from Fig. 5 that when a cosmetic is applied on the strand, a strong resurgent effect of fragrance, by repeated rubbing with a comb or the hand, can also be long-lasting for a long period of time.

Subsequently, the alcohol resistance when the fragrance-containing capsule of the present invention and etc. is blended into cosmetics was investigated. That is, an alcohol aqueous solution containing the fragrance-containing capsule obtained in the above Production Example 4-2 was prepared, in the compositions shown in the below Table 8. The value in [square brackets] in Table 8 is the blending quantity of fragrance (with respect to the total amount of the cosmetic). The evaluation results of appearance/fragrance are shown in Table 8. The results are shown in Table 8.

**[Table 8]**

| Test Example | 2-1 | 2-2 | 2-3 | 2-4 | 2-5 |
|---|---|---|---|---|---|
| Fragrance-containing capsule in Production Example 4-2 (Enclosed amount of fragrance: 15%) | 2 [0.28] | 2 [0.28] | 2 [0.28] | 2 [0.28] | 2 [0.28] |
| Ethanol | 30 | 35 | 40 | 45 | 50 |
| Ion-exchanged water | balance | balance | balance | balance | balance |
| Alcohol resistance (0°C) | A/A | A/A | A/A | A/A | A/A |
| Alcohol resistance (25°C) | A/A | A/A | A/A | A/A | A/B |
| Alcohol resistance (37°C) | A/A | A/A | A/A | A/A | A/B |
| Alcohol resistance (50°C) | A/A | A/A | A/A | A/A | A/B |

From Table 8, it is seen that when the fragrance-containing capsule of the present invention is blended into a composition including 50 mass% or more alcohol, the stability may not be maintained especially in the high-temperature region.

Accordingly, when the fragrance-containing capsule of the present invention is blended in a cosmetic, it is preferable that the blending quantity of alcohol is less than 50 mass % with respect to the total amount of the cosmetic.

Hereinafter, Formulation Examples and Blending Examples of the fragrance-containing capsule of the present invention will be listed. The present invention is not limited to these Examples.

**Formulation Example 1: Fragrance-containing capsule**

| • Core substance | |
|---|---|
| Mixed fragrance B | 20 mass % |
| Squalane | 30 |
| Decamethylcyclopentasiloxane | 10 |
| Macadamia nuts oil | 10 |
| Mortierella oil | 5 |

| • Wall material | |
|---|---|
| Crosslinked poly(methyl methacrylate) | 25 |

**< Mixed fragrance B>**

| Fruitate | 3 mass % |
|---|---|
| Ethyl 3-phenylglycidate | 3 |
| Ethyl heptanoate | 6 |
| Isoamyl butyrate | 2 |
| Ethyl butyrate | 3 |
| Isoamyl propionate | 5 |
| Butyl acetate | 3 |
| Raspberry ketone | 5 |
| Methyl amyl ketone | 4 |
| 3,5,5-trimethylhexanal | 5 |
| p-ethyl-2,2-dimethylhydrocinnamaldehyde | 1 |
| 3 -methyl-5 -phenyl-1 -pentanol | 2 |
| 2-phenylethyl alcohol | 6 |
| γ-octalactone | 3 |
| Methyl β-naphthyl ketone | 4 |
| 3-(p-tert-butylphenyl)-propanal | 2 |
| p-cresyl methyl ether | 1 |
| Patchouli alcohol | 1 |
| β-naphthyl ethyl ether | 1 |
| Thymol | 1 |
| Indole | 0.1 |
| Benzyl formate | 3 |
| Benzyl alcohol | 3 |
| Vanillin | 2 |
| Heliotropine | 1 |
| Orcinol monomethyl ether | 1 |
| Muscone | 2 |
| 3,7-dimethyl-7-methoxyoctane-2-ol | 1 |
| Cedryl methyl ether | 2 |
| Ambroxan | 3 |
| Methyl dihydrojasmonate | 20.9 |

**Formulation Example 2: Fragrance-containing capsule**

| • Core substance | |
|---|---|
| Mixed fragrance C | 25 mass % |
| Squalane | 5 |
| Liquid paraffin | 40 |
| Mortierella oil | 5 |

| • Wall material | |
|---|---|
| Crosslinked poly(methyl methacrylate) | 25 |

**< Mixed fragrance C>**

| Ethyl 3-methyl-3-phenylglycidate | 1 mass % |
|---|---|
| Ethyl caproate | 4 |
| Hexyl butyrate | 6 |
| Butyl butyrate | 4 |
| Ethyl 2-methylbutyrate | 8 |
| Ethyl propionate | 3 |
| Isoamyl acetate | 7 |
| Hexylaldehyde | 6 |
| Calone | 1 |
| Helional | 1 |
| 2-phenylethyl acetate | 2 |
| 4-isopropyl-cyclohexanol | 3 |
| Coumarin | 1 |
| Methyl anthranilate | 1 |
| Methyl salicylate | 1 |
| Benzophenone | 2 |
| Cyclamen aldehyde | 2 |
| Hydroxycitronellol | 3 |
| 2,6-dimethyl-2-heptanol | 2 |
| Anisaldehyde | 1 |
| Benzaldehyde | 2 |
| Benzyl alcohol | 3 |
| p-t-butylcyclohexyl acetate | 2 |
| 1-(2-tert-butylcyclohexyloxy)-2-butanol | 1 |
| Methyl dihydrojasmonate | 33 |

**Blending Example 1: Hair conditioner**

| Cetostearoxy PG dimethyl amine | 1.5 mass % |
|---|---|
| Dicocoyl ethyl hydroxyethyl monium methosulfate | 0.2 |
| Cetostearyl alcohol | 3.0 |
| Stearyl alcohol | 2.0 |
| Stearic acid | 0.5 |
| Glyceryl monostearate | 0.5 |
| Decamethyltetrasiloxane | 0.4 |
| Sodium lactate | 0.2 |
| Hydroxyethyl urea | 0.4 |
| Camellia raticulata seed oil | 0.1 |
| Polyquotanium 61 | 0.1 |
| Dimethicone (20 mPa·s) | 5.0 |
| Dimethiconol (10,000 mPa·s) | 1.0 |
| Mineral oil | 0.3 |
| Octyl palmitate | 0.3 |
| Steareth-4 | 0.2 |
| Sorbitol | 10.0 |
| Diglycerin | 3.0 |
| Isopulene glycol | 4.0 |
| POE(10) POP(7) dimethylether (random copolymer) | 0.2 |
| High-polymerized polyethylene glycol (Mw: 4,000,000) | 0.1 |
| Cationic starch | 0.1 |
| Capsicum tincture | 0.05 |
| Menthol | 0.1 |
| Vanillyl butyl ether | 0.02 |
| Avena fatua extract | 0.1 |
| Taurine | 1.0 |
| Phenoxyethanol | 0.4 |
| Benzyloxy ethanol | 0.3 |
| Fragrance-containing capsule (Formulation Example 2) | 2.0 |
| Fragrance | 0.6 |
| Purified water | balance |

**Blending Example 2: Hair conditioner**

| Beheroxy PG dimethyl amine | 2.5 mass % |
|---|---|
| Behenamide propyl dimethyl amine | 0.5 |
| Stearyl alcohol | 4.0 |
| Behenyl alcohol | 2.0 |
| Solid paraffin | 0.5 |
| Glyceryl monooleate | 0.4 |
| Glutamic acid | 0.6 |
| Succinic acid | 0.4 |
| L-arginine | 0.2 |
| Dimethicone (1,000 mPa•s) | 0.5 |
| Amodimethicone (1,000 mPa•s) | 1.0 |
| Dimethiconol (4,000 mPa•s) | 2.0 |
| Isocetyl isostearate | 1.0 |
| Glycerin | 5.0 |
| Isoprene glycol | 2.0 |
| POE(35) POP(40) dimethyl ether (block copolymer) | 0.3 |
| Cationic cellulose | 0.5 |
| Locust extract | 0.2 |
| Methyl paraben | 0.3 |
| Benzyl alcohol | 0.3 |
| Fragrance-containing capsule (Formulation Example 1) | 1.0 |
| Fragrance | 0.4 |
| Purified water | balance |

**Blending Example 3: Hair shampoo**

| | |
|---|---|
| Cation-modified locust bean gum (*1) | 0.6 mass % |
| Coconut oil fatty acid monoethanol amide | 2.0 |
| Dipropylene glycol | 3.0 |
| Imidazolinium betaine | 4.0 |
| Sodium N-cocoyl-N-methyl taurine | 11.0 |
| Ethylene glycol distearate | 2.7 |
| Monoglyceride oleate | 1.5 |
| Silicone emulsion (*2) | 1.5 |
| Sodium benzoate | 0.25 |
| Arginine | 0.1 |
| Fragrance-containing capsule (Formulation Example 1) | 1.0 |
| Fragrance | 0.6 |
| EDTA-2Na 2H₂O | 0.05 |
| Purified water | balance |

| | |
|---|---|
| (*1): Catinal CLB-100 (manufactured by TOHO Chemical Industry Co., Ltd.) (*2): Dimethyl silicone emulsion BY22-007 (Dimethylpolysiloxane 50 mass %) (manufactured by Dow Corning Toray Co., Ltd.) | |

**Blending Example 4: Hair shampoo**

| Cation-modified locust bean gum (*1) | 0.3 mass % |
|---|---|
| Coconut oil fatty acid monoethanol amide | 2.0 |
| Glycerin | 6.0 |
| Imidazolinium betaine | 4.0 |
| Sodium N-lauroyl-N-methyltaurine | 11.0 |
| Ethylene glycol distearate | 2.7 |
| Monoglyceride oleate | 1.5 |
| Sodium benzoate | 0.25 |
| Fragrance-containing capsule (Formulation Example 2) | 2.0 |
| Fragrance | 0.5 |
| Purified water | balance |

**Blending Example 5: Hair shampoo**

| Cation-modified locust bean gum (*1) | 0.2 mass % |
|---|---|
| Cationic cellulose | 0.5 |
| Coconut oil fatty acid monoethanol amide | 2.0 |
| Dipropylene glycol | 3.0 |
| Imidazolinium betaine | 4.0 |
| Sodium N-cocoyl-N-methyl taurine-N' -methyl taurine | 11.0 |
| Ethylene glycol distearate | 2.7 |
| Silicone emulsion (*2) | 3.0 |
| Sodium benzoate | 0.25 |
| Fragrance-containing capsule (Formulation Example 2) | 2.0 |
| Fragrance | 1.0 |
| Purified water | balance |

**Blending Example 6: Body shampoo**

| Sodium dodecane-1,2-diol acetate ether | 10.0 mass % |
|---|---|
| Sodium tetradecane-1,2-diol acetate ether | 5.0 |
| Cation-modified locust bean gum (*1) | 0.5 |
| Sodium N-cocoyl-N-methyl taurine | 5.0 |
| Coconut oil fatty acid amide propyl dimethyl amino acetate betaine | 10.0 |
| Monoglyceride oleate | 0.4 |
| Propylene glycol monolaurate | 4.0 |
| Silicone emulsion (*2) | 1.5 |
| Sodium citrate | 0.25 |
| EDTA-2Na 2H₂O | 0.05 |
| Fragrance-containing capsule (Formulation Example 1) | 0.5 |
| Fragrance | 0.9 |
| Buffer | proper quantity |
| Purified water | balance |

**Blending Example 7: Cleansing foam**

| Glycerin | 5.0 mass % |
|---|---|
| 1,3-buthylene glycol | 5.0 |
| Polyethylene glycol 400 | 10.0 |
| Beeswax | 0.4 |
| Monoglyceride oleate | 0.2 |
| Glyceryl stearate | 2.4 |
| Mixed fatty acid (C10-18) | 35.0 |
| Liquid caustic potash | 15.0 |
| Sodium N-cocoyl-N-methyl taurine | 5.0 |
| Sodium N-lauroyl-N-methyl taurine | 5.0 |
| Cation-modified locust bean gum (*1) | 0.25 |
| Silicone emulsion (*2) | 1.5 |
| Edetate salt | 0.1 |
| Iron oxide | proper quantity |
| Fragrance-containing capsule (Formulation Example 2) | 1 |
| Fragrance | 0.5 |
| Purified water | balance |

**Blending Example 8: Blusher**

| | |
|---|---|
| Kaolin | 20.0 weight part |
| Titanium dioxide | 4.2 |
| Iron oxide (red) | 0.3 |
| Red No. 202 | 0.5 |
| Ceresin | 15.0 |
| Liquid paraffin | 15.0 |
| Isopropyl myristate | 5.0 |
| Microcapsule of Blending Example 1 (solid content) | 20.0 |
| Antioxidant | proper quantity |
| Fragrance-containing capsule (Formulation Example 1) | 1.0 |
| Fragrance | 0.1 |

**Blending Example 9: Lotion**

| Glycerin | 3.0 mass % |
|---|---|
| Propylene glycol | 4.0 |
| Ethanol | 8.0 |
| Polyoxyethylene (20mol) oleyl alcohol | 0.5 |
| Citric acid | 0.01 |
| Sodium citrate | 0.1 |
| Fragrance-containing capsule (Formulation Example 1) | 0.5 |
| Fragrance | 0.05 |
| Purified water | balance |

**Blending Example 10: Cream**

| Propylene glycol | 5.0 mass % |
|---|---|
| Beeswax | 5.0 |
| Cetyl alcohol | 4.0 |
| Reduced lanolin | 5.0 |
| Squalane | 35.0 |
| Glyceride stearate | 2.0 |
| Polyoxyethylene (20mol) sorbitan monolaurate ester | 2.0 |
| Preservative | proper quantity |
| Fragrance-containing capsule (Formulation Example 2) | 1.0 |
| Fragrance | 0.1 |
| Purified water | balance |

**Blending Example 11: Facial mask**

| Polyvinyl alcohol | 15.0 mass % |
|---|---|
| Polyethylene glycol | 3.0 |
| Propylene glycol | 7.0 |
| Ethanol | 10.0 |
| Preservative | proper quantity |
| Fragrance-containing capsule (Formulation Example 2) | 1.0 |
| Fragrance | 0.1 |
| Purified water | balance |

**Blending Example 12: Scalp cosmetic**

| 1,3-buthylene glycol | 7.0 mass % |
|---|---|
| Polyethylene glycol | 5.0 |
| Ethanol | 5.0 |
| Polyoxyethylene (60mol) hydrogenated oil | 2.0 |
| Caustic potash | 0.05 |
| Carboxyvinylpolymer | 0.2 |
| 2-hexyldecyl palminate | 10.0 |
| Squalane | 5.0 |
| Beeswax | 0.5 |
| Preservative | proper quantity |
| Fragrance-containing capsule (Formulation Example 2) | 2.0 |
| Fragrance | 0.2 |
| Purified water | balance |

**Blending Example 13: Hair gel**

| Ethanol | 5.0 mass % |
|---|---|
| Diglycerin | 10.0 |
| Propylene glycol | 15.0 |
| Sorbit solution | 10.0 |
| Polyoxyethylene polyoxypropylene 2-decyl tetra decyl ether | 0.3 |
| Caustic soda | 0.1 |
| Polyvinylpyrrolidone/vinyl acetate copolymer | 4.0 |
| EDTA-2Na 2H₂O | 0.03 |
| Fragrance-containing capsule (Formulation Example 2) | 3.0 |
| Fragrance | 0.3 |
| Capsule fragrance | 2.0 |
| Purified water | balance |

**Blending Example 14: Hair water**

| Ethanol | 30.0 mass % |
|---|---|
| Dynamite glycerin | 1.0 |
| 1,3-buthylene glycol | 2.0 |
| Polyoxyethylene polyoxypropylene 2-decyl tetra decyl ether | 2.0 |
| Trehalose | 0.1 |
| Hydroxyethyl urea | 0.2 |
| Lactic acid | 0.002 |
| Sodium lactate solution (50%) | 0.12 |
| Fragrance-containing capsule (Formulation Example 1) | 2.0 |
| Fragrance | 0.6 |
| Purified water | balance |

**Blending Example 15: Deodorant spray**

| Zinc oxide | 2.0 mass % |
|---|---|
| Magnesium aluminometasilicate composite powder | 1.0 |
| Polyethylene beads | 5.0 |
| Ethanol | 5.0 |
| Benzalkonium chloride | 0.1 |
| Cyclic silicone pentamer | 0.6 |
| Sorbitan trioleate | 0.1 |
| Cetyl octanoate | 0.5 |
| Methylpolysiloxane | 1.0 |
| Menthol | 1.0 |
| L-menthyl lactate | 1.0 |
| Fragrance-containing capsule (Formulation Example 1) | 3.0 |
| Fragrance | 0.2 |
| Liquid natural gas | balance |

**Blending Example 16: Deodorant spray**

| Aluminum hydroxychloride | 0.5 mass % |
|---|---|
| Silicic anhydride | 6.0 |
| Magnesium aluminometasilicate | 1.0 |
| Niron beads | 1.0 |
| Isopropyl methylphenol | 0.5 |
| Sorbitan monooleate | 0.5 |
| Cetyl isooctanoate | 3.0 |
| Methylphenylpolysiloxane | 2.0 |
| Polyoxyethylene polyoxypropylene 2-decyl tetra decyl ether | 0.2 |
| Fragrance-containing capsule (Formulation Example 2) | 3.0 |
| Fragrance | 0.5 |
| Eucalyptus oil | 0.5 |
| Vitamin E | 0.05 |
| Menthol | 0.5 |
| Liquid natural gas | balance |

**Blending Example 17: Cologne powder spray**

| Talc | 5.0 mass % |
|---|---|
| Ethanol | 20.0 |
| 1,3-buthylene glycol | 5.0 |
| Isopropyl myristate | 1.0 |
| Sorbitan sesqui isostearate | 0.1 |
| Fragrance-containing capsule (Formulation Example 1) | 3.0 |
| Fragrance | 5.0 |
| Liquid natural gas | balance |

**Blending Example 18: Deodorant roll-on**

| Aluminium hydroxychroride | 30.0 mass % |
|---|---|
| Menthol | 0.5 |
| L-menthyl lactate | 1.0 |
| Ethanol | 40.0 |
| Eucalyptus oil | 0.01 |
| Benzalkonium chloride | 0.5 |
| Zinc oxide composite powder | 0.01 |
| Thickener | 0.05 |
| Fragrance-containing capsule (Formulation Example 1) | 5.0 |
| Purified water | balance |

**Blending Example 19: Antiperspirant deodorant lotion**

| Ethanol | 40.0 mass % |
|---|---|
| Triclosan | 0.2 |
| Zinc sulfocarbolate | 0.5 |
| Nylon powder | 4.0 |
| Glycerin | 1.0 |
| Dipropylene glycol | 1.0 |
| Potassium hydroxide | proper quantity |
| Chloro hydroxyaluminium | 0.2 |
| Lily extract | 0.2 |
| Lavender extract | 0.1 |
| White birch extract | 0.2 |
| Chamomilla recutita extract | 0.1 |
| Fragrance-containing capsule (Formulation Example 1) | 2.0 |
| Fragrance | 0.1 |
| Purified water | balance |

**Blending Example 20: Antiperspirant deodorant lotion**

| Ethanol | 45.0 mass % |
|---|---|
| Benzalkonium chloride | 0.1 |
| Zinc sulfocarbolate | 0.5 |
| Nylon powder | 1.0 |
| Crosslinked poly(methyl acrylate) powder | 4.0 |
| L-menthol | 0.1 |
| Camphor | 0.1 |
| Clove oil | 0.05 |
| Propylene glycol | 1.0 |
| Peppermint oil | 0.1 |
| Thyme extract | 0.2 |
| Aluminium chloride | 0.1 |
| Potassium hydroxide | proper quantity |
| Fragrance-containing capsule (Formulation Example 1) | 0.5 |
| Fragrance | 0.1 |
| Purified water | balance |

## Claims

1. A fragrance-containing capsule comprising;
a core substance consisting of fragrance and oil, and
a wall material formed of one or more polymers selected from the group consisting of poly(alkyl -meth-acrylates) and polystyrene,
wherein the enclosed amount of said fragrance is 1 to 60 mass %, the enclosed amount of said oil is 10 to 89 mass %, and the blending quantity of said wall material is 10 to 30 mass %, with respect to the total amount of the capsule,
wherein said fragrance is one or more selected from the group consisting of 3-octanol, 2,6-dimethyl-2-heptanol, tetrahydrogeraniol, tetrahydrolinalool, hydroxycitronellol, borneol, cedrol, patchouli alcohol, vetiverol, 4-isopropyl-cyclohexanol, 4-(isopropyl)cyclohexanemethanol, p-tert-butylcyclohexanol, o-tert-butylcyclohexanol, 1-(2-tert-butylcyclohexyloxy)-2-butanol, α,β,2,2,6-pentamethylcyclohexylpropanol, 1-(2,2,6-trimethylcyclohexyl)-3-hexanol, benzyl alcohol, 2-phenylethyl alcohol, phenoxyethyl alcohol, 1-phenylethyl alcohol, anise alcohol, 3-phenylpropyl alcohol, α,α-dimethylbenzyl carbinol, α,α-dimethylphenylethyl carbinol, phenylethylmethylethyl carbinol, 3-methyl-5-phenyl-1-pentanol, thymol, carvacrol, orcinol monomethyl ether, 3,7-dimethyl-7-methoxyoctane-2-ol, 1,8-cineole, 4-acetoxy-3-amyltetrahydropyran, cedryl methyl ether, 1-methoxycyclododecane, 1-methyl-1-methoxycyclododecane, ethoxymethyl-cyclododecyl ether, ambroxan, Gurisaruba, anisole, dimethylhydroquinone, p-cresyl methyl ether, acetanisole, dihydroanethole, diphenyl oxide, phenylethyl isoamyl ether, oxycyclododecane, ethoxymethyl-cyclododecyl ether, ambroxan, Gurisaruba, 3-phenylpropyl alcohol, α,α-dimethoctylaldehyde, nonylaldehyde, decylaldehyde, undecylaldehyde, 3,5,5-trimethylhexanal, methyloctylacetaldehyde, methylnonylacetaldehyde, hydroxycitronellal, methoxydihydrocitronellal, scentenal, benzaldehyde, phenylacetaldehyde, phenylpropylaldehyde, hydratropic aldehyde, anisaldehyde, p-methylphenylacetaldehyde, cuminaldehyde, cyclamen aldehyde, 3-(p-tert-butylphenyl)-propanal, p-ethyl-2,2-dimethylhydrocinnamaldehyde, 2-methyl-3-(p-methoxyphenyl)-propanal, 4-tert-butyl-α-methyl-hydrocinnamic aldehyde, heliotropine, helional, vanillin, ethylvanillin, octanal glycol acetal, phenylacetaldehyde dimethyl acetal, hydratropic aldehyde dimethyl acetal, phenylacetaldehyde glyceryl acetal, 2-butyl-4,4,6-trimethyl-1,3-dioxane, methyl amyl ketone, ethyl amyl ketone, methyl hexyl ketone, methyl nonyl ketone, camphor, 1-menthone, d-isomenthone, p-tert-butylcyclohexanone, 2-amylcyclopentanone, 2-heptylcyclopentanone, plicatone, 4-cyclohexyl-4-methyl-2-pentanone, 2,2,5-trimethyl-5-pentylcyclopentanone, acetophenone, p-methylacetophenone, benzyl acetone, calone, raspberry ketone, anisyl acetone, zingerone, methyl βisomenthone, p-tert-butylcyclohexanone, 2-amylcyclopentanone, 2-heptyl formate, benzyl formate, phenylethyl formate, ethyl acetate, butyl acetate, isoamyl acetate, hexyl acetate, isononyl acetate, l-menthyl acetate, n-bornyl acetate, isobornyl acetate, p-t-butylcyclohexyl acetate, o-t-butylcyclohexyl acetate, benzyl acetate, 2-phenylethyl acetate, styrallyl acetate, anisyl acetate, p-cresyl acetate, heliotropyl acetate, cedryl acetate, vetiveryl acetate, decahydro-β-naphthyl acetate, ethyl propionate, isoamyl propionate, benzyl propionate, ethyl butyrate, ethyl 2-methylbutyrate, butyl butyrate, isoamyl butyrate, hexyl butyrate, benzyl butyrate, benzyl isobutyrate, phenylethyl isobutyrate, phenoxyethyl isobutyrate, ethyl isovalerate, propyl valerate, benzyl isovalerate, phenylethyl isovalerate, ethyl caproate, ethyl heptanoate, ethyl 2-methylpentanoate, ethyl octanoate, ethyl ketopropionate, isoamyl ketopropionate, ethyl acetoacetate, ethyl levulinate, methyl benzoate, benzyl benzoate, phenylethyl benzoate, methyl phenylacetate, methyl salicylate, methyl anisate, methyl anthranilate, methyl dihydrojasmonate, ethyl 3-methyl-3-phenylglycidate, ethyl 3-phenylglycidate, fructone, fraistone, fruitate, γ-octalactone, coumarin, muscone, cyclopentadecanone, cyclopentadecanolide, and indole wherein said oil is one or more selected from the group consisting of liquid paraffin, ozokerite, squalane, pristane, paraffin, squalene, petrolatum, macadamia nut oil, isopropyl myristate , cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceryl tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, mortierella oil, linear polysiloxanes, and cyclic polysiloxanes.

2. According to a fragrance-containing capsule of Claim 1,
wherein the volume average particle diameter D50 of said capsule is 1 to 100 µm.

3. A cosmetic comprising;
a fragrance-containing capsules according to Claim 1 or 2.

4. The cosmetic according to Claim 3,
wherein the blending quantity of alcohol is less than 50 mass %.

## Patentansprüche

1. Duftstoff enthaltende Kapsel, umfassend;
eine Kernsubstanz, bestehend aus Duftstoff und Öl, und
ein Wandmaterial, gebildet aus einem oder mehreren Polymeren, ausgewählt aus der Gruppe bestehend aus Poly(alkylmethacrylaten) und Polystyrol,
wobei die eingeschlossene Menge des Duftstoffes 1 bis 60 Massen-% beträgt, die eingeschlossene Menge des Öls 10 bis 89 Massen-% beträgt und die Mischungsmenge des Wandmaterials 10 bis 30 Massen-% beträgt, bezogen auf die Gesamtmenge der Kapsel,
wobei der Duftstoff einer oder mehrere ist, ausgewählt aus der Gruppe bestehend aus 3-Octanol, 2,6-Dimethyl-2-heptanol, Tetrahydrogeraniol, Tetrahydrolinalool, Hydroxycitronellol, Borneol, Cedrol, Patchuli-Alkohol, Vetiverol, 4-Isopropylcyclohexanol, 4-(Isopropyl)cyclohexanmethanol, p-tert-Butylcyclohexanol, o-tert-Butylcyclohexanol, 1-(2-tert-Butylcyclohexyloxy)-2-butanol, α,β,2,2,6-Pentamethylcyclohexylpropanol, 1-(2,2,6-Trimethylcyclohexyl)-3-hexanol, Benzylalkohol, 2-Phenylethylalkohol, Phenoxyethylalkohol, 1-Phenylethylalkohol, Anisalkohol, 3-Phenylpropylalkohol, α,α-Dimethylbenzylcarbinol, α,α-Dimethylphenylethylcarbinol, Phenylethylmethylethylcarbinol, 3-Methyl-5-phenyl-1-pentanol, Thymol, Carvacrol, Orcinolmonomethylether, 3,7-Dimethyl-7-methoxyoctan-2-ol, 1,8-Cineol, 4-Acetoxy-3-amyltetrahydropyran, Cedrylmethylether, 1-Methoxycyclododecan, 1-Methyl-1-methoxycyclododecan, Ethoxymethylcyclododecylether, Ambroxan, Gurisaruba, Anisol, Dimethylhydrochinon, p-Kresylmethylether, Acetanisol, Dihydroanethol, Diphenyloxid, Phenylethylisoamylether, Oxycyclododecan, Ethoxymethylcyclododecylether, Ambroxan, Gurisaruba, 3-Phenylpropylalkohol, α,α-Dimethoctylaldehyd, Nonylaldehyd, Decylaldehyd, Undecylaldehyd, 3,5,5-Trimethylhexanal, Methyloctylacetaldehyd, Methylnonylacetaldehyd, Hydroxycitronellal, Methoxydihydrocitronellal, Scentenal, Benzaldehyd, Phenylacetaldehyd, Phenylpropylaldehyd, Hydratropaldehyd, Anisaldehyd, p-Methylphenylacetaldehyd, Cuminaldehyd, Cyclamenaldehyd, 3-(p-tert-Butylphenyl)propanal, p-Ethyl-2,2-dimethylhydrozimtaldehyd, 2-Methyl-3-(p-methoxyphenyl)propanal, 4-tert-Butyl-α-methylhydrozimtaldehyd, Heliotropin, Helional, Vanillin, Ethylvanillin, Octanalglykolacetal, Phenylacetaldehyddimethylacetal, Hydratropinaldehyddimethylacetal, Phenylacetaldehydglycerylacetal, 2-Butyl-4,4,6-trimethyl-1,3-dioxan, Methylamylketon, Ethylamylketon, Methylhexylketon, Methylnonylketon, Campher, 1-Menthon, d-Isomenthon, p-tert-Butylcyclohexanon, 2-Amylcyclopentanon, 2-Heptylcyclopentanon, Plicaton, 4-Cyclohexyl-4-methyl-2-pentanon, 2,2,5-Trimethyl-5-pentylcyclopentanon, Acetophenon, p-Methylacetophenon, Benzylaceton, Calon, Himbeerketon, Anisylaceton, Zingeron, Methyl-β-isomenthon, p-tert-Butylcyclohexanon, 2-Amylcyclopentanon, 2-Heptylformiat, Benzylformiat, Phenylethylformiat, Ethylacetat, Butylacetat, Isoamylacetat, Hexylacetat, Isononylacetat, 1-Menthylacetat, n-Bornylacetat, Isobornylacetat, p-tert-Butylcyclohexylacetat, o-tert-Butylcyclohexylacetat, Benzylacetat, 2-Phenylethylacetat, Styrallylacetat, Anisylacetat, p-Kresylacetat, Heliotropylacetat, Cedrylacetat, Vetiverylacetat, Decahydro-β-naphthylacetat, Ethylpropionat, Isoamylpropionat, Benzylpropionat, Ethylbutyrat, Ethyl-2-methylbutyrat, Butylbutyrat, Isoamylbutyrat, Hexylbutyrat, Benzylbutyrat, Benzylisobutyrat, Phenylethylisobutyrat, Phenoxyethylisobutyrat, Ehylisovalerat, Propylvalerat, Benzylisovalerat, Phenylethylisovalerat, Ethylcaproat, Ethylheptanoat, Ethyl-2-methylpentanoat, Ethyloctanoat, Ethylketopropionat, Isoamylketopropionat, Ethylacetoacetat, Ethyllevulinat, Methylbenzoat, Benzylbenzoat, Phenylethylbenzoat, Methylphenylacetat, Methylsalicylat, Methylanisat, Methylanthranilat, Methyldihydrojasmonat, Ethyl-3-methyl-3-phenylglycidat, Ethyl-3-phenylglycidat, Fructon, Fraiston, Fruitat, γ-Octalacton, Cumarin, Muscon, Cyclopentadecanon, Cyclopentadecanolid und Indol, wobei das Öl eines oder mehrere ist, ausgewählt aus der Gruppe bestehend aus flüssigem Paraffin, Ozokerit, Squalan, Pristan, Paraffin, Squalen, Vaseline, Macadamianussöl, Isopropylmyristat, Cetyloctanoat, Octyldodecylmyristat, Isopropylpalmitat, Butylstearat, Hexyllaurat, Myristylmyristat, Decyloleat, Hexyldecyldimethyloctanoat, Cetyllactat, Myristyllactat, acetyliertes Lanolin, Isocetylstearat, Isocetylisostearat, Cholesteryl-12-hydroxystearat, Ethylenglykol-di-2-ethylhexanoat, Dipentaerythrit-Fettsäureester, N-Alkylglykolmonoisostearat, Neopentylglykoldicaprat, Diisostearylmalat, Glyceryl-di-2-heptylundecanoat, Trimethylolpropan-tri-2-ethylhexanoat, Trimethylolpropantriisostearat, Pentaerythrit-tetra-2-ethylhexanoat, Glyceryl-tri-2-ethylhexanoat, Glyceryltrioctanoat, Glyceryltriisopalmitat, Trimethylolpropantriisostearat, Cetyl-2-ethylhexanoat, 2-Ethylhexylpalmitat, Glyceryltrimyristat, Glyceryltri-2-heptylundecanoat, Rizinusöl-Fettsäuremethylester, Oleyloleat, Acetoglycerid, 2-Heptylundecylpalmitat, Diisobutyladipat, N-Lauroyl-L-glutaminsäure-2-octyldodecylester, Di-2-heptylundecyladipat, Ethyllaurat, Di-2-ethylhexylsebacat, 2-Hexyldecylmyristat, 2-Hexyldecylpalmitat, 2-Hexyldecyladipat, Diisopropylsebacat, 2-Ehylhexylsuccinat, Triethylcitrat, Mortierella-Öl, lineare Polysiloxane und cyclische Polysiloxane.

2. Nach einer Duftstoff enthaltenden Kapsel nach Anspruch 1,
wobei der volumengemittelte Partikeldurchmesser D50 der Kapsel 1 bis 100 µm beträgt.

3. Kosmetik, umfassend;
eine Duftstoff enthaltende Kapsel nach Anspruch 1 oder 2.

4. Kosmetik nach Anspruch 3,
wobei die Mischungsmenge an Alkohol weniger als 50 Massen-% beträgt.

## Revendications

1. Une capsule contenant un parfum comprenant:
une substance principale comprenant un parfum et une huile, et
un matériau de paroi formé d'un ou plusieurs polymères choisis parmi le groupe constitué par des poly(méth)acrylates d'alkyle et du polystyrène,
la quantité enfermée comprenant ledit parfum est de 1 à 60 % en masse, la quantité enfermée de ladite huile est de 10 à 89 % en masse et la quantité de mélange du matériau de paroi est de 10 à 30 % en masse par rapport à la quantité totale de la capsule,
dans laquelle ledit parfum est un ou plusieurs choisis parmi le groupe constitué par le 3-octanol, 2,6-diméthyl-2-heptanol, tétrahydrogéraniol, tétrahydrolinalol, hydroxycitronellol, bornéol, cédrol, l'alcool patchouli, vétiverol, 4-isopropylcyclohexanol, 4-(isopropyl)cyclohexaneméthanol, p-tert-butylcyclohexanol, o-tert-butylcyclohexanol, 1-(2-tert-butylcyclohexyloxy)-2-butanol, α,β,2,2,6-pentaméthylcyclohexylpropanol, 1-(2,2,6-triméthylcyclohexyl)-3-hexanol, alcool benzylique, alcool 2-phényléthylique, alcool phénoxyéthylique, alcool 1-phényléthylique, alcool d'anis, alcool 3-phénylpropylique, α,α-ou diméthylbenzylcarbinol, α, α-diméthylphényléthylcarbinol, phényléthylméthyléthylcarbinol, 3-méthyl-5-phényl-1-pentanol, thymol, carvacrol, l'éther monométhylique de l'orcinol, le 3,7-diméthyl-7-méthoxyoctane-2-ol, le 1,8-cinéole, le 4-acétoxy-3-amyltétrahydropyrane, l'éther méthylique de cedryle, 1-méthoxycyclododécane, 1-méthyl-1-méthoxycyclododécane, l'éther éthoxyméthylique de cyclododécyl, ambroxyde, Gurisaruba, anisole, diméthylhydroquinone, p-crésylméthyléther, acétanisole, dihydroanethole, diphényle oxyde, éther phényléthyl-iso-amylique, oxycyclododécane, éther éthoxyméthyl-cyclododécylique, ambroxyde, Gurisaruba, alcool 3-phénylpropylique, α,α-diméthoctylaldéhyde, nonylaldéhyde, décylaldéhyde, undécylaldéhyde, 3,5,5-triméthylhexanal, méthyloctylacétaldéhyde, méthylnonylacétaldéhyde, hydroxycitronellal, méthoxydihydrocitronellal, scentental, benzaldéhyde, phénylacétaldéhyde, phénylpropylaldéhyde, aldéhyde hydratropique, anisaldéhyde, p-méthylphénylacétaldéhyde, cuminaldéhyde, aldéhyde de cyclamen, 3-(p-tertbutylphényl)-propanal, p-éthyl-2,2-diméthylhydrocinnamaldéhyde, 2-méthyl-3-(p-méthoxyphényl)-propanal, aldéhyde 4-tert-butyl-α-méthyl-hydrocinnamique, héliotropine, hélional, vanilline, éthylvanilline, acétal glycolique d'octanal, acétal diméthylique de l'aldéhyde phénylacétique, acétal diméthylique de l'aldéhyde hydratropique, acétal glycérylique de l'aldéhyde phénylacétique, 2-butyl-4,4,6-triméthyl-1,3-dioxane, méthylamylcétone, éthylamylcétone, méthylhexylecétone, méthylnonylcétone, camphre, 1-menthone, d-isoménthone, p-tert-butylcyclohexanone, 2-amylcyclopentanone, 2-heptylcyclopentanone, plicatone, 4-cyclohexyl-4-méthyl-2-pentanone, 2,2,5-triméthyl-5-pentylcyclopentanone, acétophénone, p-méthylacétophénone, acétone de benzyle, calone, cétone de framboise, acétone d'anisyle, zingerone, méthyl-β-isomenthone, p-tertbutylcyclohexanone, 2-amylcyclopentanone, formiate de 2-hept, formiate de benzyle, formiate de phényléthyle, acétate d'éthyle, acétate de butyle, acétate d'isoamyle, acétate d'hexyle, acétate d'isononyle, acétate de 1-menthyle, acétate de n-bornyle, acétate d'isobornyle, acétate de p-t-butylcyclohexyle, acétate de o-t-butylcyclohexyle, acétate de benzyle, acétate de 2-phényléthyle, acétate de styralyle, acétate d'anisyle, acétate de p-crésyle, acétate d'héliotropyle, acétate de cedryle, acétate de vétiveryle, acétate de décahydro-β-naphtyle, propionate d'éthyle, propionate d'isoamyle, propionate de benzyle, butyrate d'éthyle, 2-méthylbutyrate d'éthyle, butyrate de butyle, butyrate d'isoamyle, butyrate d'hexyle, butyrate de benzyle, isobutyrate de benzyle, isobutyrate de phényléthyle, isobutyrate de phénoxyéthyle, isovalérate d'éthyle, valérate de propyle, isovalérate de benzyle, isovalérate de phényléthyle, caproate d'éthyle, heptanoate d'éthyle, 2-méthylpentanoate d'éthyle, octanoate d'éthyle, cétopropionate d'éthyle, cétopropionate d'isoamyle, acétoacétate d'éthyle, lévulinate d'éthyle, benzoate de méthyle, benzoate de benzyle, benzoate de phényléthyle, phénylacétate de méthyle, salicylate de méthyle, anisate de méthyle, anthranilate de méthyle, dihydrojasmonate de méthyle, éthyle 3-méthyl-3-phénylglycidate, éthyl-3-phénylglycidate, fructone, fraistone, fruitate, γ-octalactone, coumarine, muscone, cyclopentadécanone, cyclopentadécanolide, et indole dans laquelle ladite huile est une ou plusieurs choisies parmi le groupe constitué par la paraffine liquide, l'ozokérite, le squalane, le pristane, paraffine, squalène, pétrolatum, huile de noix de macadamia, myristate d'isopropyle, octanoate de cétyle, myristate d'octyldodécyle, palmitate d'isopropyle, stéarate de butyle, laurate d'hexyle, myristate de myristyle, oléate de décyle, diméthyloctanoate d'hexyldécyle, lactate de cétyle, lactate de myristyle, lanoline acétylée, stéarate d'isocétyle, isostéarate d'isocétyle, 12-hydroxystéarate de cholestérol, di-2-éthylhexanoate d'éthylène glycol, ester d'acide gras de dipentaérythritol, monoisostéarate de N-alkylglycol, dicaprate de néopentyle glycol, malate de diisostéaryle, di-2-heptylundécanoate de glycéryle, tri-2-éthylhexanoate de triméthylolpropane, triisostéarate de triméthylolpropane, tétra-2-éthylhexanoate de pentaérythritol, tri-2-ethylhexanoate de glycéryle, trioctanoate de glycéryle, triisopalmitate de glycéryle, triisostéarate de triméthylolpropane, 2-éthylhexanoate de cétyle, palmitate de 2-éthylhexyle, trimyristate de glycéryle, tri-2-heptylundecanoate de glycéryle, ester méthylique d'acide gras d'huile de ricin, oleate d'oléyle, acétoglycéride, palmitate de 2-heptylundécyle, adipate de diisobutyle, ester 2-octyldodécylique de l'acide n-lauroyl-L-glutamique, adipate de di-2-heptylundécyl, le laurate d'éthyle, le sébacate de di-2-éthylhexyle, le myristate de 2-hexyldécyle, le palmitate de 2-hexyldécyle, l'adipate de 2-hexyldécyle, le sébacate de diisopropyle, le succinate de 2-éthylhexyle, le citrate de triéthyle, l'huile de mortierella, polysiloxanes linéaires et polysiloxanes cycliques.

2. Capsule contenant un parfum selon la revendication 1,
dans laquelle la taille de particule moyenne en volume D50 de ladite capsule est de 1 à 100 µm.

3. Un cosmétique comprenant :
capsules contenant un parfum selon l'une quelconque des revendications 1 ou 2.

4. Le cosmétique selon la revendication 3,
dans lequel la quantité d'alcool mélangée est inférieure à 50% en masse.
